# EUROPEAN PATENT APPLICATION

(11) **EP 2 452 628 A1**
(43) Date of publication of application: **16.05.2012**
(21) Application number: 11188636.2
(22) Date of filing: 10.11.2011
(51) Int. Cl.: A61B 10/02

(54) **Skin biopsy device**

(30) Priority: 12.11.2010 TR 201009467
(71) Applicant: Adnan Menderes Universitesi, 09010 Aydin (TR)
(72) Inventor: Bozdag, Ali Dogan, 09010 AYDIN (TR); Bozdag, Kubra Eren, 09010 AYDIN (TR)
(74) Representative: Dericioglu, Ekin

(57) **Abstract**

The present invention relates to a skin biopsy device (100) which performs biopsy cutaneously and subcutaneously. With the inventive biopsy device (100), the tissue wanted to be performed biopsy is enabled to be removed with vacuum. Also the base of the tissue wanted to be performed biopsy is cut without harming the adjacent tissues. The biopsy device (100) is comprised of at least one body (1) which enables the tissue piece wanted to be performed biopsy to be pulled with vacuum and a housing (2) having a mechanism which enables the tissue piece to be cut by being fixed on the body (1).

## Description

### Field of the Invention

The present invention relates to a skin biopsy device which performs biopsy cutaneously and subcutaneously.

### Background of the Invention

In the state of the art, skin biopsy devices used in skin biopsy is tubular and has a sharp structure like knife at the end of the tube. The device is pressed and rotated on the skin wherein the biopsy is to be performed by being held between two fingers. Thus, by means of the sharp structure at the end part of the device, a cylindrical cut involving the skin and subcutaneous tissue is made. The said cylindrical tissue needs to be held and pulled with tools such as forceps, needle in order that its base is to be cut. This pulling can cause difficulty in histopathological examination by causing the tissue to be harmed. The pulled tissue is cut from its base with tools such as bistoury and scissors. But this process creates risks such as not being able to cut the base flatly and cutting the adjacent tissues.

In some embodiments known in the state of the art, the tissue is held and pulled with a device comprising structures like tabs going inside the tissue or hook-like structures. In these cases, the tissue can be ruptured or harmed. Also in cases wherein the said device is applied to the fragile tissues, it is hard to pull the tissue upwards. And in some cases, since the distance between the hooks and the sharp structure is definite, the said distance can be inadequate in order to remove the lesions going deep.

United States Patent document no US2009018467, an application known in the state of the art, discloses four hook-like structures which hold the tissue. While the said hook-like structures are stuck into the tissue and pulling it upwards, the base of the tissue is cut with the knife therein.

In the United States patent document no US2006253046, an application known in the state of the art, while the tissue biopsy is being performed, the tissue is grabbed with the small tabs going inside the tissue and held upwards and its base is cut.

In the United States patent document no US200526261603, an application known in the state of the art, the tissue which is cut cylindrically with a biopsy device is held and pulled with forceps, needle or a hook, and in the meantime the tissue is removed by being cut with a bistoury, knife or scissors.

United States patent document no US2008039740, an application known in the state of the art, discloses cylindrical knife and a semilunar shaped knife therein which takes cut from the base of the tissue.

United States patent document no US5014717, an application known in the state of the art, discloses a punch biopsy apparatus. One end of the said apparatus has trocar support opposite to the end of the punch, and the opposite end has a cannula-aiming device which is shaped with a finger model for finger surgery.

United States patent document no US2004049127, an application known in the state of the art, discloses a tissue perforation device and a method. The device preferably comprises a housing having a housing pass-through, a penetrator securely and sealably positioned so that the penetrator device passes through the housing pass-through, and a vacuum system.

Tissue biopsy devices in the state of the art do not have the features to pull the tissue upwards by creating vacuum without harming and to cut the base of the tissue flatly without harming the adjacent tissues.

### Summary of the Invention

The objective of the present invention is to realize a skin biopsy device which performs biopsy without harming the tissue.

A further objective of the present invention is to realize a skin biopsy device which enables the tissue wanted to be performed biopsy to be removed with vacuum.

Another objective of the present invention is to realize a skin biopsy device which cuts the base of the tissue wanted to be performed biopsy without harming the adjacent tissues.

A further objective of the present invention is to realize a transparent skin biopsy device.

### Detailed Description of the Invention

"A Skin Biopsy Device" realized to fulfill the objectives of the present invention is illustrated in the accompanying figures, in which:

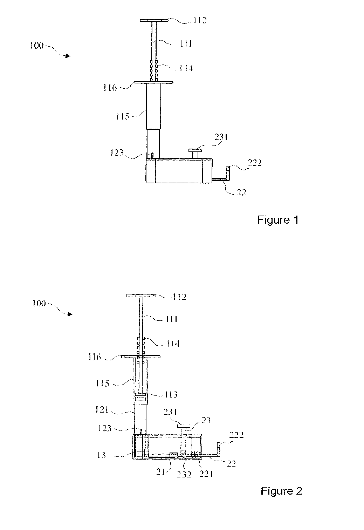

Figure 1 is the side view of the biopsy device.
Figure 2 is the side section view of the biopsy device.

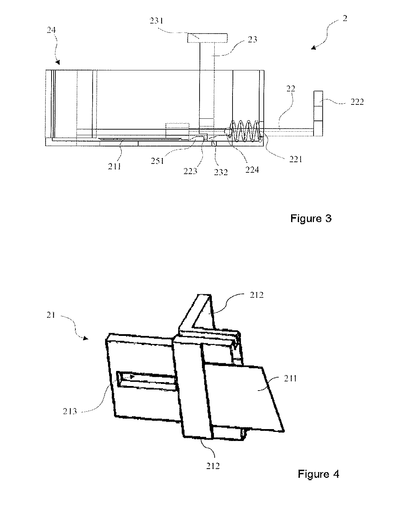

Figure 3 is the side section view of the housing.
Figure 4 is the perspective view of the cutter.

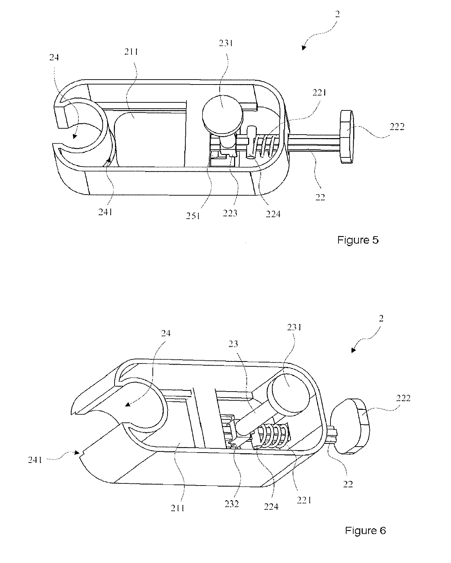

Figure 5 is the perspective view of inside the housing.
Figure 6 is another perspective view of inside the housing.

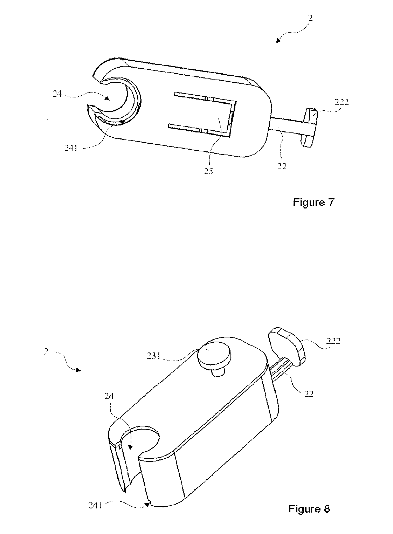

Figure 7 is the perspective view of the housing from below.
Figure 8 is the perspective view of the housing from above.

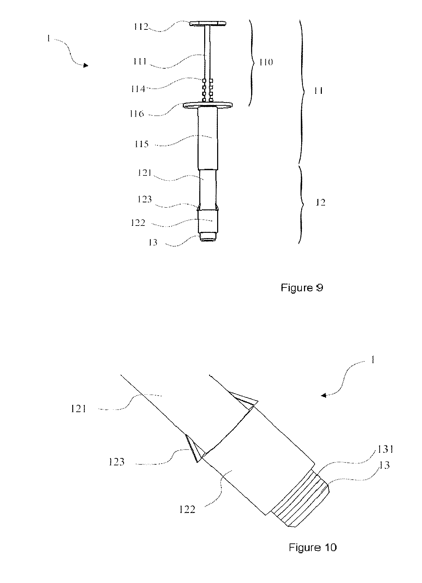

Figure 9 is the perspective view of the body.
Figure 10 is the perspective view of the tube and the cutting base.

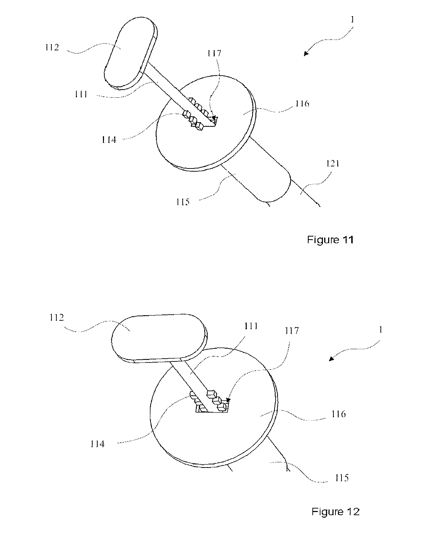

Figure 11 is the perspective view of the moment when the body is attached to the protruding opening from above.
Figure 12 is the perspective view of the moment when the body is detached from the protruding opening from above.

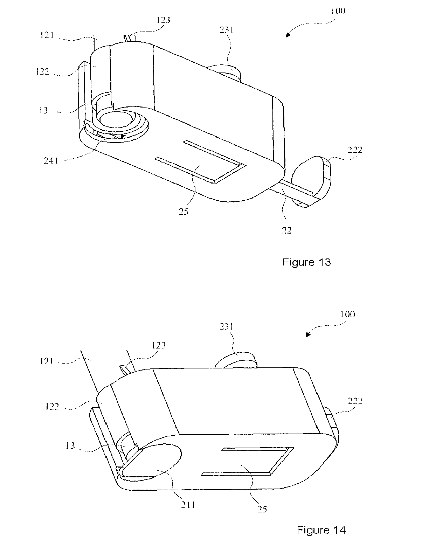

Figure 13 is the from below perspective view of the cocking lever of the biopsy device when the lever is acock.
Figure 14 is the from below perspective view of the moment when the cocking lever of the biopsy device is released.

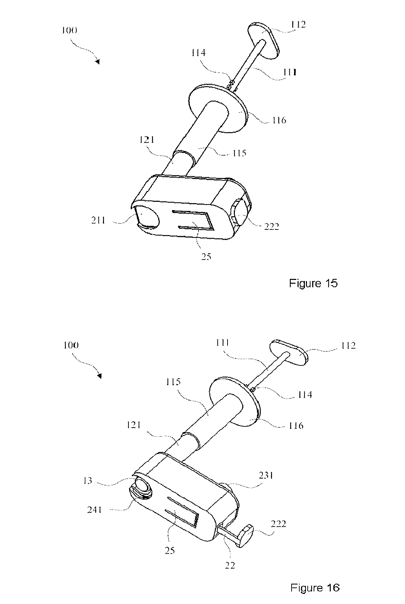

Figure 15 is another from below perspective view of the moment when the cocking lever of the biopsy device is released.
Figure 16 is a further from below perspective view of the moment when the cocking lever of the biopsy device is released.

The components given in the figures are numbered and the numbers refer to the following:
100. Biopsy device
1. Body
   11. Injection syringe
      110. Piston
      111. Piston rod
      112. Pressure surface
      113. Sealing member
      114. Protrusion
      115. Injection syringe body
      116. Support surface
      117. Opening
   12. Tube
      121. Narrow part
      122. Wide part
      123. Protrusion
   13. Cutting base
2. Housing
   Cutter
   211. Knife
   212. Holder
   213. Channel
   22. Cocking lever
   221. Spring
   222. Cocking member
   223. Protrusion
   224. Support
   23. Trigger
   231. Pushing surface
   232. Protrusion
   24. Body opening
   241. Knife opening
   25. Latch
   251. Strut

The inventive biopsy device (100) is comprised of at least one body (1) which enables the tissue piece wanted to be performed biopsy to be pulled with vacuum and a housing (2) having a mechanism which enables the tissue piece to be cut by being fixed on the body (I).

### The body (1) comprises

- at least one injection syringe (11) which creates vacuum in order to pull up the tissue which is cut,
- at least one tube (12) to which the housing (2) is connected through the body (1), and which extends at the same direction with the injection syringe (11) at the end part ofthe injection syringe (11),
- at least one cutting base (13) which is located at the close end of the tube (12) to the tissue and enables the lateral surfaces of the tissue to be cut.

In preferred embodiment of the invention, the tube (12) and the cutting base (13) which is located on the close part of the tube (12) to the tissue have a hollow cylindrical structure.

In preferred embodiment of the invention, the cutting base (13) which is located on the close part of the tube (12) to the skin has a structure expanding from skin to the tube (12).

In preferred embodiment of the invention, there are lines (131) on the cutting base (13) which enable the skin insertion depth to be observed. The lines (131) are preferably circular.

The injection syringe (11) comprises
- at least one injection syringe body (115),
- at least one piston (110) which creates vacuum by moving inside the injection syringe (115).

In preferred embodiment of the invention, the injection syringe (115) has a hollow cylindrical structure.

The piston (110) comprises
- at least one piston rod (111) which transmits the pushing/pulling force applied by the operator,
- at least one pressure surface (112) which is located at the end of the piston rod (111) not close to the tissue, on which the force is applied at pushing/pulling status,
- at least one sealing member (113) which prevents the air passage during the movement of the piston rod (111) inside the injection syringe (115) by being positioned at the end of the piston rod (111) close to the tissue,
- at least one protrusion (114) which limits the movement of the piston rod (111) on the injection syringe (115) by being positioned with intervals on the piston rod (111).

In the preferred embodiment of the present invention, preferably six protrusions (114) are placed on the piston rod (111) with intervals.

The injection body (115) comprises
- at least one support surface (116) which is located at the end of the injection syringe (115) not close to the tissue being vertical to the injection syringe body (115), and wherein the operator gets support during applying force on the pressure surface (112),
- at least one opening (117) which is located in the middle of the support surface (116), allows the movement of the piston rod (111) when it is at the same level with the protrusion (114).

In the preferred embodiment of the present invention the support surface (116) placed at the end of the injection syringe body (115) not close to the tissue has circular structure.

The tube (12) located at the end of the injection syringe (11) close to the tissue comprises
- at least one narrow part (121) wherein the housing (2) is engaged to the body (1),
- at least one wide part (122) under the narrow part, located in the part close to the skin and has the width which can enable the housing (2) to be fixed on the body (1), and
- at least one protrusion (123) on the narrow part (121), extending from the narrow part (121) to the wide part and which enables the housing (2) to stay fixed on the wide part (122).

One end of the protrusion (123) is fixed to the narrow part (121) whereas the other end is unattached. The end which is unattached contacts the upper surface of the housing (2) upon the housing (2) being fixed on the wide part (122).

The body (1) is transparent except the cutting base (13) located at its end part. The body (1) being transparent enables the part to be performed biopsy on the skin to be seen and the cutting base (13) to be placed depending on the status of the part to be performed biopsy.

The housing (2) comprises
- at least one cutter (21) which enables the tissue piece the side surfaces of which are cut by the cutting base (13) to be cut from its lower surface,
- at least one cocking lever (22) which is connected to the end of the cutter (21) that does not cut the tissue and which locks by fixing the cutter (21) at a certain position,
- at least one trigger (23) one end of which is located inside the housing (2), and the other end of which extends from the housing (2) to outside and which enables the cocking lever (22) to be released,
- at least one body opening (24) wherein the narrow part (121) can pass through, and
- at least one moving latch (25) which is located at the housing base (2).

In the preferred embodiment of the invention, the trigger (23) is cylindrical.

In preferred embodiment of the invention, the body opening (24) extends between the upper and lower surfaces of the housing (2) in a semilunar shape.

The latch (25) is fixed to the base of the housing (2) from its one edge. The fixed edge is preferably fixed to the part of the housing towards the body opening (24), and its other edges stay unattached. In the preferred embodiment of the invention, the latch (25) is rectangular.

The latch (25) comprises on its surface facing the housing (2) at least one strut (251) which enables the latch (25) to move downwards with the movement of the cocking lever (22).

The cutter (21) located in the housing (2) comprises
- at least one knife (211) which enables the lower part of the tissue piece to be cut,
- at least one holder (212) which enables the knife (211) to be held,
- at least one channel (213) which is located on the holder (212), wherein the strut (251) passes through.

One end of the cocking lever (22) is connected to the cutter (21) from the side wherein the cutting is not performed, and the other end extends outside from the housing (2).

The cocking lever (22) comprises
- at least one spring (221) which is located on the part close to the end of the cocking lever (22) extending outside the housing, one end of which is unattached and the other end of which contacts the housing (2) inside,
- at least one cocking member (222) which is located at the end of the cocking lever (22) outside the housing (2) and which provides holding surface to the operator during pulling,
- at least one protrusion (223) which is located on the lower surface of the cocking lever (22), which pushes the latch (25) downwards upon the cocking lever (22) being pulled and contacting the strut (251), which is fixed upon the contact with the strut (251) being ended and the latch (25) moving upwards, and
- at least one support (224) which is located on the upper surface of the cocking lever (22), which compresses the spring (221) upon the strut (251) and the protrusion (223) being locked to each other.

Upon the movement of the trigger (23) towards the housing (2) and contacting the latch (25), the protrusion (223) is released from the strut (252) and the cocking lever (22) moves towards the tissue the lower surface of which is to be cut.

The trigger (23) comprises
- at least one pushing surface (231) which enables the trigger (23) to be pushed by being fixed to its end outside the housing (2),
- at least one protrusion (232) which is fixed to the end of the trigger (23) inside the housing and has an opening wide enough for the support (224) to pass through.

In the preferred embodiment of the invention, the protrusion (232) is in the shape of a horseshoe.

Pushing force is applied on the trigger (23) upon enabling the end part of the cutting base (13) inserted to the tissue and the end of the knife (211) close to the body opening (24) to be aligned. With this move, the protrusion (232) contacts the latch (25), upon the protrusion (223) being released from the strut (251), the knife (211) moves towards the tissue and cuts the lower surface of the tissue.

The body opening (24) which has the width enough to wrap the body (1) and is located on the housing (2) comprises a knife opening (241) which has the sizes to make it possible for knife (211) to move by passing between the cutting base (12) and the skin from the part of the housing (2) close to the skin.

The housing (2) is transparent except the knife (211) and the spring (221) located therein. The housing (2) being transparent enables the alignment of the end of the cutting base (13) and the knife (211) therein to be seen.

While the biopsy is being performed, first the cutting base (13) is pressed and rotated on the skin to be performed biopsy. The insertion depth of the cutting base (13) to the skin and the depth of the cut are determined from the lines (131) on the cutting base (13). After the cut is performed in a desired depth, the piston rod (111) is rotated such that it can pass through the opening (117) on the support surface (116) and it is pulled from the pressure surface (112). By means of the vacuum, the cut tissue is kept inside the tube (12). When the desired height is reached, the protrusions (114) are enabled to be attached to the support surface (116) and the piston rod (111) to remain at a stable height by the piston rod (111) being rotated again.

Upon the skin and the subcutaneous tissue to be performed biopsy being pulled inside the body (1), the housing (2) which is previously assembled is fixed on the body (1).

In the assembly of the housing (2), first the protrusion (223) is enabled to be locked to the strut (251) by the cocking lever (22) being pulled outside the housing (2) by being held from its cocking surface (222). Synchronously with the protrusion (224) being locked to the strut (251), the support (224) compresses the spring (221) with the inner wall of the housing (2). By this means the assembly of the cocking lever (22) is realized and at the same time the knife (211) is enabled to be released to the body opening (24).

After the side surfaces of the tissue is cut and the piston (110) pulls the tissue upwards with vacuum, the body (1) is attached with the housing (2) which is assembled.

Upon the body opening (24) is passed over the narrow part of the body (1) the housing (2) is attached to the body (1). Upon being attached the operator compresses on the housing (2) from the narrow part (121) to the wide part (122). By this means the housing (2) is enabled to move towards the skin on the tube (12).When the movement of the housing (2) from the narrow part (121) to the wide part (122) is completed, the protrusion contacts the upper surface of the housing (2) and prevents the housing (2) from moving upwards and fixes on the body (1).

The cocking lever (22), which is cocked, is activated upon the pressure is applied on the pushing surface (231) of the trigger (23) which is outside the housing (2). As a result of the pressure applied on the pushing surface (231) of the trigger (23), the protrusion (232) which is located at the other end of the trigger (23) and the latch (25) on the housing (2) base are pushed and the protrusion (224) is released from the strut (251). Thus, the knife (211) which is acock is enabled to be released. As a result of the knife (211) being released and moving towards the tissue, the lower part of the tissue is cut and the sample is taken for the biopsy.

Within the scope of these basic concepts, it is possible to develop various embodiments of the inventive biopsy device (100). The invention can not be limited to the examples described herein; it is essentially as defined in the claims.

## Claims

1. A skin biopsy device (100) **characterized by** at least one body (1) which enables the tissue piece wanted to be performed biopsy to be pulled with vacuum, and has
- at least one injection syringe (11) which creates vacuum in order to pull up the tissue which is cut,
- at least one tube (12) which extends at the same direction with the injection syringe (11) at the end ofthe injection syringe (11),
- at least one cutting base (13) which is located at the close end of the tube (12) to the tissue and enables the lateral surfaces ofthe tissue to be cut, and housing (2) which enables the tissue piece to be cut by being fixed on the body (1), has
- at least one cutter (21) which enables the tissue part the side surfaces of which are cut by the cutting base (13) to be cut from its lower surface,
- at least one cocking lever (22) which is connected to the end of the cutter (21) that does not cut the tissue and which locks by fixing the cutter (21) at a certain position,
- at least one trigger (23) which enables the cocking lever (22) to be released,
- at least one body opening (24) wherein the narrow part (121) can pass through, and
- at least one moving latch (25).

2. A skin biopsy device (100) according to claim 1, **characterized by** cutting base (13) which has a hollow cylindrical structure and has lines (131) thereon enabling the insertion depth to the skin to be observed.

3. A biopsy device (100) according to any one of the preceding claims, **characterized by** injection syringe (11) which has at least one injection syringe body (115) having a hollow cylindrical structure and at least one piston (110) creating vacuum by moving inside the injection syringe body (115).

4. A skin biopsy device (100) according to claim 3, **characterized by** at least one piston (110) which has
- at least one piston rod (111) which transmits the pushing/pulling force applied by the operator,
- at least one pressure surface (112) which is located at the end of the piston rod (111) not close to the tissue, on which the force is applied at pushing/pulling status,
- at least one sealing member (113) which prevents the air passage during the movement of the piston rod (111) inside the injection syringe (115) by being positioned at the end of the piston rod (111) close to the tissue,
- at least one protrusion (114) which limits the movement of the piston rod on the injection syringe body (115) by being placed with intervals on the piston rod (111).

5. A skin biopsy device (100) according to claim 3 or 4, **characterized by** injection syringe body (115) which has
- at least one support surface (116) which is located at the end of the injection syringe (115) not close to the tissue being vertical to the injection syringe body (115), and wherein the operator gets support during applying force on the pressure surface (112),
- at least one opening (117) which is located in the middle of the support surface (116), allows the movement of the piston rod (111) when it is aligned with the protrusion (114).

6. A skin biopsy device (100) according to any one of the preceding claims, **characterized by** tube (12) which has
- at least one narrow part (121) wherein the housing (2) is engaged to the body (1),
- at least one wide part (122) under the narrow part, located in the part close to the skin and has the width which can enable the housing (2) to be fixed on the body (1), and
- at least one protrusion (123) on the narrow part (121), extending from the narrow part (121) to the wide part and which enables the housing (2) to stay fixed on the wide part (122).

7. A skin biopsy device (100) according to any one of the preceding claims, **characterized by** body (1) which is transparent except the cutting base (13) located on its end part.

8. A skin biopsy device (100) according to any one of the preceding claims, **characterized by** body opening (24) which extends in a semilunar shape between the upper and lower surfaces of the housing (2).

9. A skin biopsy device (100) according to any one of the preceding claims, **characterized by** latch (25) in a rectangular shape which is fixed to the housing (2) from its one edge, and the other edges of which are unattached.

10. A skin biopsy device (100) according to claim 9, **characterized by** latch (25) having at least one strut (251) which is located on its surface facing the housing (2), and enables the latch (25) to move downwards with the movement of cocking lever(22).

11. A skin biopsy device (100) according to claim 10, **characterized by** cutter (21) which has
- at least one knife (211) which enables the lower part of the tissue part to be cut,
- at least one holder (212) which enables the knife (211) to be held,
- at least one channel (213) located on the holder (212), wherein the strut (251) passes through.

12. A skin biopsy device (100) according to the any one of the preceding claims, **characterized by** cocking lever (22) one end of which is connected to the cutter (21) from the side wherein the cutting is not performed, the other end extends outside from the housing (2).

13. A skin biopsy device (100) according to claim 12, **characterized by** cocking lever (22) having
- at least one spring (221) which is located on the part close to the end of the cocking lever (22) extending outside the housing, one end of which is unattached and the other end of which contacts the housing (2) inside,
- at least one cocking member (222) which is located at the end of the cocking lever (22) outside the housing (2) and which provides holding surface to the operator during pulling,
- at least one protrusion (223) which is located on the lower surface of the cocking lever (22), which pushes the latch (25) downwards upon the cocking lever (22) being pulled and contacting the strut (251), which is fixed upon the contact with the strut (251) being ended and the latch (25) moving upwards, and
- at least one support (224) which is located on the upper surface of the cocking lever (22), which compresses the spring (221) upon the strut (251) and the protrusion (223) being locked to each other.

14. A skin biopsy device (100) according to claim 13, **characterized by** trigger (23) having
- at least one pushing surface (231) which enables the trigger (23) to be pushed by being fixed to its end outside the housing (2),
- at least one protrusion (232) which is fixed to the end of the trigger (23) inside the housing and has an opening wide enough for the support (224) to pass through

15. A skin biopsy device (100) according to any one of the claims 11 to 14, **characterized by** body opening (24) having a knife opening (241) which has the sizes to make it possible for knife (211) to move by passing between the cutting base (12) and the skin from the part of the housing (2) close to the skin
